(19) Europäisches Patentamt
European Patent Office
Office européen des brevets

(11) Veröffentlichungsnummer: **0 132 230**
**B1**

(12) # EUROPÄISCHE PATENTSCHRIFT

(45) Veröffentlichungstag der Patentschrift: **05.10.88**

(51) Int. Cl.⁴: **C 07 D 239/00** // C07D251/00, C07D249/14

(21) Anmeldenummer: **84810346.1**

(22) Anmeldetag: **13.07.84**

(54) **Verfahren zur Herstellung von Sulfonylharnstoffen.**

(30) Priorität: **18.07.83 CH 3928/83**
**10.04.84 CH 1804/84**

(43) Veröffentlichungstag der Anmeldung: **23.01.85 Patentblatt 85/4**

(45) Bekanntmachung des Hinweises auf die Patenterteilung: **05.10.88 Patentblatt 88/40**

(84) Benannte Vertragsstaaten: **AT BE CH DE FR GB IT LI NL SE**

(56) Entgegenhaltungen:
**EP - A - 0 044 807**
**EP - A - 0 044 808**
**EP - A - 0 044 809**
**EP - A - 0 070 802**
**EP - A - 0 094 790**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Töpfl, Werner, Dr., Dorneckstrasse 68, CH-4143 Dornach (CH)**

EP 0 132 230 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der vorliegenden Erfindung ist ein Verfahren zur Herstellung von Sulfonylharnstoffen der allgemeinen Formel I

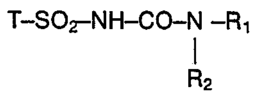 (I)

worin

$R_1$ H, $C_1$–$C_4$-Alkyl

$R_2$ 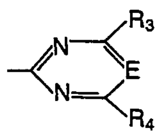 oder 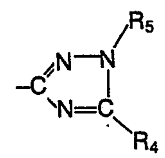 ,

E =N– oder =CH–,

$R_3$ $C_1$–$C_4$-Alkyl, $C_1$–$C_4$-Alkoxy oder Halogen,

$R_4$ $C_1$–$C_4$-Alkyl, $C_3$–$C_6$-Cycloalkyl, $C_1$–$C_4$-Alkoxy, Halogen, $C_1$–$C_4$-Alkoxy-$C_1$–$C_4$-alkyl, Halogen-$C_1$–$C_4$-alkyl oder Halogen-$C_1$–$C_4$-alkoxy,

$R_5$ H oder $C_1$–$C_4$-Alkyl

T einen substituierten Phenylrest

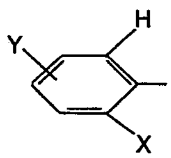 oder 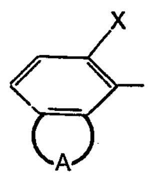 ,

Y H oder Halogen

X H, Halogen, Nitro, $C_1$–$C_4$-Alkyl, Halogen-$C_1$–$C_4$-alkyl, $C_2$–$C_4$-Alkenyl, Halogen-$C_2$–$C_4$-alkenyl, $C_2$–$C_4$-Alkinyl, $C_1$–$C_4$-Alkoxy, Halogen-$C_1$–$C_4$-alkoxy, $C_1$–$C_4$-Alkoxy-$C_1$–$C_4$-alkoxy, $C_1$–$C_4$-Alkylthio, $C_1$–$C_4$-Alkylsulfinyl, $C_1$–$C_4$-Alkylsulfonyl, Halogen-$C_1$–$C_4$-alkylthio, $C_1$–$C_4$-Alkylsulfonyloxy, Phenyl, unsubstituiertes Phenylsulfonyloxy oder ein- oder gegebenenfalls mehrfach $C_1$–$C_4$-alkylsubstituiertes Phenylsulfonyloxy oder $C_1$–$C_4$-Dialkylsulfamoyl bedeuten und

A für ein gegebenenfalls substituiertes 3- oder 4 atomiges Brückenglied steht, das 1 oder 2 Heteroatome enthält und zusammen mit den es bindenden Kohlenstoffatomen einen nichtaromatischen 5- bis 6-gliedrigen Heterocyclus bildet, wobei zwei Sauerstoffatome durch mindestens ein Kohlenstoffatom getrennt sind und Sauerstoff und Schwefelatome nur dann miteinander verbunden sind, wenn der Schwefel als -SO- oder -$SO_2$-Gruppe vorliegt, durch Umsetzung eines Sulfonamids der Formel II

$$T-SO_2-NH_2 \quad II$$

in Gegenwart einer Base mit Diphenylcarbonat zu einem Salz eines Phenylcarbamats der Formel III

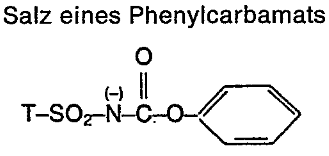 $Me^+$ (III),

worin $Me^+$ ein Kation des Natriums oder des Kaliums oder eines tertiären Amins bedeutet, Überführung dieses Salzes in das freie Phenylcarbamat der Formel IIIa

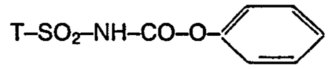 (IIIa)

und dessen weitere Umsetzung mit einem Amin der Formel IV,

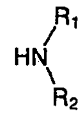 (IV),

in der Weise, dass man die Umsetzung des Sulfonamids der Formel II mit dem Diphenylcarbonat unter Ausschluss von Wasser in einem aprotischen Lösungsmittel bei 0–30 °C durchführt und ohne Isolierung eines Zwischenprodukts anschliessend aus dem gebildeten Salz eines Phenylcarbamates der Formel III durch Zugabe einer wasserfreien Säure das Phenylcarbamat der Formel IIIa freisetzt und durch Umsetzung mit einem Amin der Formel IV bei 20–150 °C in einen Sulfonylharnstoff der Formel I überführt.

Sulfonylharnstoffe der Formel I besitzen eine ausgezeichnete herbizide Wirkung und können daher vorteilhaft als herbizide Mittel verwendet werden. Sulfonylharnstoffe der Formel I, ihre Herstellung und Verwendung sind beispielsweise in den US-Patentschriften Nr. 4 127 405, 4 169 719, 4 238 621 und in der EP-A-0 084 020 beschrieben.

Es ist bekannt, dass der p-Toluolsulfonyl-butylharnstoff aus p-Toluolsulfonyl-phenylcarbamat und Butylamin herzustellen ist [Jap. Patent Application No.: 33-13484, C.A. 1962/I 7218 g].

Es ist ferner bekannt, dass aus dem Natriumsalz von p-Toluolsulfonamid und Diphenylcarbonat das Natriumsalz von p-Toluolsulfonylphenylcarbamat entsteht, aus welchem das N-p-Toluolsulfonylphenylcarbamat durch Säurezusatz freigesetzt und isoliert werden kann. [Jap. Patent Application No.: 33-13483, C.A. 1962/I 7218 f].

Die Herstellung des hydrolyseempfindlichen Sulfonylphenylcarbamats als Zwischenprodukt wird nach diesem Verfahren nach einem umständlichen Aufarbeitungsverfahren (Extraktion mit Äther) in Anwesenheit von Wasser isoliert. Die Ausbeute an Phenyl-p-toluolsulfonylcarbamat beträgt nur 50% d.Th. Die Ausbeute am Endprodukt 1-(p-Toluolsulfonyl)-3-butylharnstoff beträgt 47% d.Th., bezogen auf das eingesetzte p-Toluolsulfonamid.

Weiterhin ist aus den europäischen Patentanmeldungen EP-A-0 044 807, EP-A-0 044 808 und EP-A-0 044 809 bekannt, herbizid wirksame Sulfonylharnstoffe durch Umsetzung von Phenylcarbamaten mit heterocyclischen Aminen herzustellen. Die Phenylcarbamate werden analog zu der vorgenannten Literaturstelle aus einem Sulfonamid und Diphenylcarbonat hergestellt. Bedingt durch die niedrige Basizität der Sulfonamide, ist

eine direkte Umsetzung der Edukte (Sulfonamid und Diphenylcarbonat) nicht möglich. In einer ersten Reaktionsstufe wird unter Einwirkung starker Basen, wie Natriumhydrid, aus dem Sulfonamid das entsprechende Salz hergestellt, welches dann mit dem Diphenylcarbonat zum Carbamatsalz reagiert. Durch Neutralisieren dieses Salzes im wässrigen Medium ist das Phenylcarbamat erhältlich, welches schliesslich mit dem heterocyclischen Amin zum Sulfonamid umgesetzt wird.

Die Herstellung von Sulfonylharnstoffen der Formel I erweist sich nach dieser Methode wegen der angeführten Nachteile als technisch uninteressant.

Nach dem erfindungsgemässen Verfahren werden bevorzugt Sulfonamide der Formel IIa

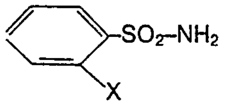

IIa

eingesetzt, in welchen X die oben angegebene Bedeutung hat.

Geeignete Ausgangsprodukte zur Durchführung des Verfahrens sind beispielsweise folgende Sulfonamide der Formel II

2-Fluor-benzolsulfonamid
2-Chlor-benzolsulfonamid
2-Nitro-benzolsulfonamid
2-Methyl-benzolsulfonamid
2-Trifluormethyl-benzolsulfonamid
2-Methoxy-benzolsulfonamid
2-Difluormethoxy-benzolsulfonamid
2-(2′-Chloräthoxy)-benzolsulfonamid
2-(2′-Methoxy-äthoxy)-benzolsulfonamid
2-n-Propylthio-benzolsulfonamid
2-Methylsulfonyl-benzolsulfonamid
2-n-Propylsulfonyl-benzolsulfonamid
2-Methylsulfonyloxy-benzylsulfonamid
2-n-Propylsulfonyloxy-benzolsulfonamid
2-Dimethylsulfamoyl-benzolsulfonamid
2,3-Dihydro-benzfuran-7-yl-sulfonamid.

Als Amine der Formel IV werden nach dem erfindungsgemässen Verfahren

2-Amino-4-methyl-6-methoxy-s-triazin
2-Amino-4-methyl-6-methoxy-pyrimidin
2-Amino-4-methyl-6-difluormethoxy-pyrimidin
2-Amino-4,6-dimethoxy-pyrimidin
2-Amino-4,6-dimethoxy-s-triazin
2-Amino-4,6-dimethyl-pyrimidin
2-Amino-4-methoxy-6-äthoxy-s-triazin
2-Amino-4-dimethylamino-6-methoxy-s-triazin
2-Amino-4-cyclopropyl-6-methoxy-s-triazin
2-Amino-4-methoxy-6-methoxy-methyl-pyrimidin
2-Amino-4-methoxy-6-chlorpyrimidin
2-Amino-4-methoxy-6-chlormethyl-pyrimidin
3-Amino-1,5-dimethyl-1,2,4-triazol und
3-Amino-5-methoxy-1-methyl-1,2,4-triazol

bevorzugt, wenn beim eingesetzten Arylsulfonamid der Formel II Y Wasserstoff ist.

Zur Durchführung des erfindungsgemässen Verfahrens bestehen mehrere Varianten. Nach der ersten Variante wird das Sulfonamid der Formel II zunächst mit der äquivalenten Menge einer Base in ein Alkalisalz, wie z.B. Natrium- oder Kaliumsalz oder in ein Salz eines geeigneten tertiären Amins übergeführt. Die anschliessende Aufarbeitung des Reaktionsgutes wird so gestaltet, dass das resultierende Salz zum Schluss als Lösung oder als Suspension in einem aprotischen Lösungsmittel unter Ausschluss von Wasser vorliegt.

Die folgenden Umsetzungen nach dem erfindungsgemässen Verfahren werden dann in einem aprotischen Lösungsmittel in Abwesenheit von Wasser durchgeführt.

Als aprotische Lösungsmittel eignen sich z.B. Acetonitril, Tetrahydrofuran, Dioxan, 1,2-Dimethoxyäthan, Dimethylformamid, N,N-Dimethyl-acetamid, N-Methylpyrrolidon oder auch Gemische derselben. Als Lösungsmittel eignet sich insbesondere Acetonitril.

Zur Überführung des Sulfonamids der Formel II in sein Salz stehen mehrere Möglichkeiten zur Verfügung.

Zur Herstellung der Alkalisalze kann das Sulfonamid der Formel II zunächst mit einem Alkalihydroxyd in einem Lösungsmittel, z.B. Alkohol umgesetzt werden, das resultierende Alkalisalz kann nach Zugabe von einem zweiten Lösungsmittel wie z.B. Toluol und nach Verdampfen der Lösungsmittel als wasserfreier Rückstand gewonnen werden. Dieser kann gegebenenfalls im Vakuum nachgetrocknet werden.

Als Alkalihydroxyde eignen sich Natrium- oder Kaliumhydroxyd. Das Kaliumhydroxyd ist als Alkalihydroxyd bevorzugt.

Das Natrium- und Kaliumsalz des Sulfonamids der Formel II kann auch durch Umsetzung des freien Sulfonamids mit einem Natrium- oder Kaliumalkoholat in alkoholischer Lösung analog erhalten werden. Das resultierende Alkalisalz des Sulfonamids der allgemeinen Formel II wird nach allfälliger Trocknung anschliessend in einem aprotischen Lösungsmittel suspendiert.

Die tertiären Ammoniumsalze der Sulfonamide der allgemeinen Formel II können als wasserfreie Lösungen durch Vermischung äquivalenter Mengen des Sulfonamids und eines geeigneten tertiären Amins in einem aprotischen Lösungsmittel hergestellt und unmittelbar weiterverwendet werden. Die tertiären Amine müssen hierzu eine zur Salzbildung mit dem Sulfonamid ausreichende Basizität aufweisen. Als tertiäre Amine eignen sich zur Salzbildung z.B. das 1,8-Diazabicyclo(5,4,0)undec-7-en oder das 1,5-Diazabicyclo-(4,3,0)non-5-en. Diese salzbildenden tertiären Amine sind käuflich und können nach der Umsetzung wiedergewonnen werden.

Die so hergestellte Suspension oder Lösung des Alkali- oder tertiären Ammoniumsalzes des Sulfonamids kann nach dem erfindungsmässigen Verfahren nach mehreren Methoden in den entsprechenden Sulfonylharnstoff übergeführt werden. Nach einer bevorzugten, in einem einzigen Reaktionsgefäss durchführbaren Methode wird das in einem aprotischen Lösungsmittel suspendierte oder gelöste Salz mit äquivalenter Menge Diphenylcarbonat vermischt und 1 bis 12 Stunden

lang bei 0–30 °C Temperatur gerührt, wobei die erwünschte Reaktion zum Sulfocarbamat-Salz abläuft. Die Gesamtmenge des Sulfocarbamats wird daraus durch Zugabe von äquivalenter Menge einer geeigneten wasserfreien Säure, wie z.B. Methansulfonsäure oder Chlorwasserstoff, gegebenenfalls gelöst, bei 0–30 °C freigesetzt. Methansulfonsäure ist als wasserfreie Säure bevorzugt. Der Einsatz von Chlorwasserstoff, gelöst in Acetonitril, gilt ebenfalls als bevorzugt. Die resultierende Sulfocarbamat-Lösung wird mit äquivalenter Menge des heterocyclischen Amins zum Sulfonylharnstoff umgesetzt, wobei die Temperatur des Reaktionsguts von anfangs 20 °C bis zum Kochpunkt des Lösungsmittels, jedoch maximal 150 °C erhöht wird. Die Zugabe der Säure und des heterocyclischen Amins kann dabei auch gleichzeitig erfolgen. Nach einer anderen Methode, wird das heterocyclische Amin in einem zweiten Reaktionsgefäss, in einem aprotischen Lösungsmittel durch Säurezugabe in sein entsprechendes Salz übergeführt und als solches eingesetzt. Das suspendierte oder gelöste Sulfocarbamatsalz wird dann zum suspendierten oder gelösten Aminsalz zwischen Raumtemperatur und Kochpunkt des Lösungsmittels gegeben. Nach beendeter Zugabe des Sulfocarbamats wird das Reaktionsgut zur Vervollkommnung der Umsetzung wie vorhin beschrieben, erwärmt.

Nach einer zweiten, besonders bevorzugten Variante zur Durchführung des erfindungsgemässen Verfahrens wird das zur Salzbildung mit dem Sulfonamid eingesetzte tert. Amin unter Ausschluss von Wasser in ein vorgelegtes, aus dem Sulfonamid, aus Diphenylcarbonat und aus dem aprotischen Lösungsmittel bestehende Gemisch getropft und das Phenylcarbamat-Salz gebildet. Die Weiterverarbeitung dieses Zwischenproduktes kann nach einer der vorhin beschriebenen Methoden erfolgen.

Die Aufarbeitung des Reaktionsgutes erfolgt am einfachsten, wenn der resultierende Sulfonylharnstoff beim Abkühlen auf 0 bis 20 °C rein auskristallisiert. Das Reaktionsprodukt wird in diesem Fall nach Filtrieren, Nachwaschen und Trocknen rein erhalten. Nach Aufarbeitung der Mutterlauge können das Lösungsmittel, das als Nebenprodukt entstandene Phenol sowie das gegebenenfalls eingesetzte salzbildende Amin in reiner Form abgetrennt werden. Wenn die Kristallisation des Sulfonylharnstoffs nur im unbefriedigenden Masse oder gar nicht stattfindet, kann zunächst das Lösungsmittel verdampft, dann gegebenenfalls das als Nebenprodukt entstandene Phenol unter vermindertem Druck abdestilliert werden und der Destillationsrückstand mit Wasser und mit einem mit Wasser nicht mischbaren Lösungsmittel, wie z.B. Toluol oder Cyclohexan, warm aufgenommen werden, worauf der Sulfonylharnstoff aus der in der Wärme abgetrennten Lösungsmittelschicht beim Abkühlen auskristallisiert. Das Produkt wird filtriert, nachgewaschen und getrocknet. Durch destillative Aufarbeitung der Mutterlauge, sowie der abgetrennten wässrigen Schicht können beide Lösungsmittel, das als Nebenprodukt entstandene Phenol, sowie das gegebenenfalls verwendete salzbildende Amin in reiner Form isoliert werden. Die auf das eingesetzte Sulfonamid bezogene Ausbeute beträgt 50–80% d.Th. über die beiden Stufen.

Das erfindungsgemässe Verfahren ist gegenüber dem Stand der Technik vorteilhaft, weil es ermöglicht, die hydrolyseempfindlichen Sulfonylphenylcarbamate unter Ausschluss von Wasser mit verbesserter Ausbeute herzustellen und die gesamte Reaktionsfolge in einem Reaktionsgefäss durchzuführen.

Nach der besonders bevorzugten Variante des erfindungsgemässen Verfahrens, nach welcher das tert. Amin in ein vorgelegtes, aus dem Sulfonamid, aus Diphenylcarbonat und aus dem Lösungsmittel bestehende Gemisch gegeben wird, können durch empfindliche Gruppen substituierte Sulfonamide der Formel II, welche unter den Bedingungen anderer Verfahrensvarianten kein erwünschtes Produkt liefern, in die entsprechenden Sulfonylharnstoffe übergeführt werden. Als solche Sulfonamide gelten die 2-$C_1$–$C_4$-Alkylsulfonyloxy, unsubstituierte Phenylsulfonyloxy oder ein- oder gegebenenfalls mehrfach $C_1$–$C_4$-alkylsubstituierte Phenylsulfonyloxybenzolsulfonamide und die 2(2-Halogen-$C_1$–$C_4$-alkoxy)-benzolsulfonamide. Durch Anwendung dieser besonders vorteilhaften Verfahrensvariante werden allgemein noch höhere Ausbeuten an Sulfonylharnstoffen erzielt, als nach der anderen Verfahrensvariante.

Beispiel 1:

Herstellung von N-[2-(N,N-Dimethyl-sulfamoyl)-phenyl-sulfonyl]-N′-(4-chlormethyl-6-methoxy-pyrimidin-2-yl)-harnstoff

26,4 g 2-(N,N-Dimethyl-sulfamoyl)-benzolsulfonamid (0,1 Mol) werden in 100 ml Acetonitril suspendiert, 15,7 g 1,8-Diazabicyclo(5,4,0)undec-7-en DBU (0,1 Mol) zugegeben und 1 Stunde bei Raumtemperatur gerührt. 21,4 g Diphenylcarbonat (0,1 Mol) werden hinzugefügt und 1 Stunde gerührt. Nach 15 Stunden Stehen werden 9,7 g Methansulfonsäure (0,1 Mol) in der Kälte zugetropft. Danach werden 17,3 g 2-Amino-4-chlormethyl-6-methoxy-pyrimidin (0,1 Mol) zugegeben und die Suspension 1 Stunde unter Rückfluss erhitzt. Dabei entsteht zunächst eine Lösung aus der sich nach kurzer Zeit das Produkt kristallin abscheidet. Das Reaktionsgut wird abgekühlt und das Produkt abgenutscht. Es wird 32 g leichtgelbliches Produkt vom Fp. 214–216° (Zers.) erhalten. Ausbeute 70% d.Th.

Beispiel 2:

N-(2-Trifluormethyl-phenylsulfonyl)-N′-(4-methoxy-6-methoxymethyl-pyrimidin-2-yl)-4′-harstoff

11,3 g (0,05 Mol) 2-Trifluormethyl-benzolsulfonamid, 30 g Methanol und 5,8 g (0,05 Mol) K-tert.-Butylat werden in einem Rührkolben vermischt und das Lösungsmittelgemisch wird im Vakuum verdampft. Der Rückstand von 13,0 g, das K-Salz des Sulfonamids, wird in 20 g Dioxan suspendiert, unter Rühren mit 10,8 g (0,05 Mol) Diphenylcarbonat versetzt und 12 Stunden bei Raumtemperatur

gerührt. Dabei entsteht eine Suspension des Kalium-Salzes vom Phenylcarbamat. 1,8 g (0,05 Mol) HCl-Gas wird in die kalte Lösung von 8,5 g (0,05 Mol) 2-Amino-4-methoxy-6-methoxymethyl-pyrimidin in 25 g Dioxan unter Rühren eingeleitet, wobei das Hydrochlorid des Amins als Suspension entsteht.

Hierzu wird bei derselben Temperatur während 30 Minuten die vorher beschriebene Suspension des K-Salzes vom Phenylcarbamat gegeben. Das Reaktionsgut wird weitere 30 Minuten bei Rückflusstemperatur gehalten und anschliessend wird das Dioxan bei Normaldruck und das Phenol in Vakuum abdestilliert. Der aus festem KCl und aus einem öligen organischen Teil bestehende Rückstand wird mit 25 g Toluol bei 60° verdünnt, das KCl mit 20 g Wasser gelöst und Schichten bei 60 °C werden getrennt.

Aus der toluolischen Schicht in der Kälte umkristallisierte Produkt wird abfiltriert, nachgewaschen und getrocknet. Ausbeute 15 g, 71% d.Th.

Beispiel 3:
Herstellung von N-(2-Methylsulfonyloxy-phenylsulfonyl)-N′-(4-chlormethyl-6-methoxy-pyrimidin-2-yl)-harnstoff

25,1 g 2-Methylsulfonyloxy-benzolsulfonamid (0,1 Mol), 21,4 g Diphenylcarbonat (0,1 Mol) und 50 ml Acetonitril werden verrührt. Zu diesem vorgelegten Gemisch werden bei 0–5 °C 15,7 g 1,8-Diazabicyclo-(5,4,0)undec-7-en DBU (0,1 Mol) während 10 Minuten, unter Rühren zugetropft. Die resultierende Lösung wird 15 Stunden lang bei Raumtemperatur stehen gelassen. Zu der abgekühlten Lösung werden 9,7 g Methansulfonsäure (0,1 Mol) bei 0–5 °C während 10 Minuten unter Rühren zugetropft. Anschliessend werden noch 17,3 g 2-Amino-4-chlormethyl-6-methoxy-pyrimidin (0,1 Mol) zugegeben und die resultierende Suspension wird eine Stunde lang unter Rückfluss gekocht, wobei eine klare Lösung entsteht. Diese wird abgekühlt, das dabei auskristallisierende Produkt wird abgenutscht. Es wird 29,5 g Produkt vom Fp. 177–179 °C erhalten. Ausbeute 65% d.Th.

**Patentansprüche**

1. Verfahren zur Herstellung von Sulfonylharnstoffen der allgemeinen Formel I

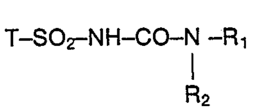 (I)

worin
$R_1$ H, $C_1$–$C_4$-Alkyl

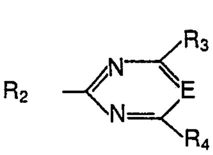 oder 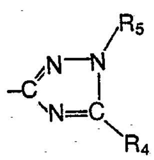 ,

E =N– oder =CH–,
$R_3$ $C_1$–$C_4$-Alkyl, $C_1$–$C_4$-Alkoxy oder Halogen,
$R_4$ $C_1$–$C_4$-Alkyl, $C_3$–$C_6$-Cycloalkyl, $C_1$–$C_4$-Alkoxy, Halogen, $C_1$–$C_4$-Alkoxy-$C_1$–$C_4$-alkyl, Halogen-$C_1$–$C_4$-alkyl oder Halogen-$C_1$–$C_4$-alkoxy,
$R_5$ H oder $C_1$–$C_4$-Alkyl,
T einen substituierten Phenylrest

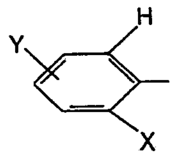 oder 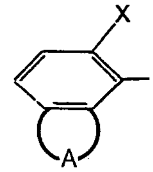 ,

Y H oder Halogen,
X H, Halogen, Nitro, $C_1$–$C_4$-Alkyl, Halogen-$C_1$–$C_4$-alkyl, $C_2$–$C_4$-Alkenyl, Halogen-$C_2$–$C_4$-alkenyl, $C_2$–$C_4$-Alkinyl, $C_1$–$C_4$-Alkoxy, Halogen-$C_1$–$C_4$-alkoxy, $C_1$–$C_4$-Alkoxy-$C_1$–$C_4$-alkoxy, $C_1$–$C_4$-Alkylthio, $C_1$–$C_4$-Alkylsulfinyl, $C_1$–$C_4$-Alkylsulfonyl, Halogen-$C_1$–$C_4$-alkylthio, $C_1$–$C_4$-Alkylsulfonyloxy, Phenyl, unsubstituiertes Phenylsulfonyloxy oder ein- oder gegebenenfalls mehrfach $C_1$–$C_4$-alkyl-substituiertes Phenylsulfonyloxy oder $C_1$–$C_4$-Dialkylsulfamoyl bedeuten und

A für ein gegebenenfalls substituiertes 3- oder 4 atomiges Brückenglied steht, das 1 oder 2 Heteroatome enthält und zusammen mit den es bindenden Kohlenstoffatomen einen nichtaromatischen 5- bis 6-gliedrigen Heterocyclus bildet, wobei zwei Sauerstoffatome durch mindestens ein Kohlenstoffatom getrennt sind und Sauerstoff und Schwefelatome nur dann miteinander verbunden sind, wenn der Schwefel als –SO- oder –$SO_2$-Gruppe vorliegt, durch Umsetzung eines Sulfonamids der Formel II

$$T{-}SO_2{-}NH_2 \qquad \text{II}$$

in Gegenwart einer Base mit Diphenylcarbonat zu einem Salz eines Phenylcarbamats der Formel III

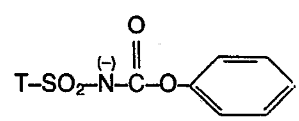 $Me^+$ (III),

worin $Me^+$ ein Kation des Natriums oder des Kaliums oder eines tertiären Amins bedeutet, Überführung dieses Salzes in das freie Phenylcarbamat der Formel IIIa

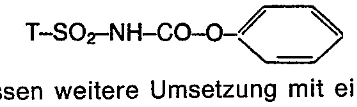 (IIIa)

und dessen weitere Umsetzung mit einem Amin der Formel IV

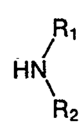 (IV),

dadurch gekennzeichnet, dass man die Umsetzung des Sulfonamids der Formel II mit dem Diphenylcarbonat unter Ausschluss von Wasser in einem aprotischen Lösungsmittel bei 0–30 °C durchführt und ohne Isolierung eines Zwischenprodukts anschliessend aus dem gebildeten Salz eines Phenylcarbamates der Formel III durch Zugabe einer wasserfreien Säure das Phenylcarbamat der Formel IIIa freisetzt und durch Umsetzung mit einem Amin der Formel IV bei 20–150 °C in einen Sulfonylharnstoff der Formel I überführt.

2. Verfahren gemäss Anspruch 1 zur Herstellung von Sulfonylharnstoffen der allgemeinen Formel I

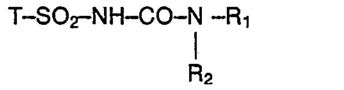 (I)

worin $R_1$ und $R_2$ die im Anspruch 1 angegebenen Bedeutungen haben

T einen substituierten Phenylrest

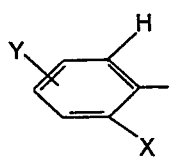 oder 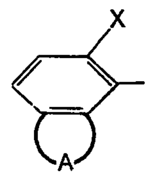

und

X H, Halogen, $C_1$–$C_4$-Alkyl, Halogen-$C_1$–$C_4$-alkyl, $C_1$–$C_4$-Alkenyl, Halogen-$C_2$–$C_4$-alkenyl, $C_2$–$C_4$-Alkinyl, $C_1$–$C_4$-Alkoxy, Halogen-$C_1$–$C_4$-alkoxy, $C_1$–$C_4$-Alkylthio, $C_1$–$C_4$-Alkylsulfinyl, $C_1$–$C_4$-Alkylsulfonyl, Halogen-$C_1$–$C_4$-alkylthio oder $C_1$–$C_4$-Dialkylsulfamoyl bedeutet und A die im Anspruch 1 angegebene Bedeutung hat.

3. Verfahren gemäss Anspruch 1, dadurch gekennzeichnet, dass man folgende Sulfonamide der Formel II einsetzt:
2-Fluor-benzolsulfonamid
2-Chlor-benzolsulfonamid
2-Nitro-benzolsulfonamid
2-Methyl-benzolsulfonamid
2-Trifluormethyl-benzolsulfonamid
2-Methoxy-benzolsulfonamid
2-Difluormethoxy-benzolsulfonamid
2-(2′-Chloräthoxy)-benzolsulfonamid
2-(2′-Methoxy-äthoxy)-benzolsulfonamid
2-n-Propylthio-benzolsulfonamid
2-Methylsulfonyl-benzolsulfonamid
2-n-Propylsulfonyl-benzolsulfonamid
2-Methylsulfonyloxy-benzylsulfonamid
2-n-Propylsulfonyloxy-benzolsulfonamid
2-Dimethylsulfamoyl-benzolsulfonamid
2,3-Dihydro-benzfuran-7-yl-sulfonamid.

4. Verfahren gemäss Anspruch 1, dadurch gekennzeichnet, dass man Sulfonamide der Formel IIa

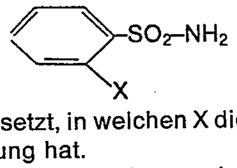 IIa

bevorzugt einsetzt, in welchen X die oben angegebene Bedeutung hat.

5. Verfahren gemäss Anspruch 1, dadurch gekennzeichnet, dass man Amine der Formel IV verwendet, in welchen $R_1$ Wasserstoff und $R_2$ ein 4-Methyl-6-methoxy-s-triazin-2-yl oder 4-Methyl-6-methoxy-pyrimidin-2-yl oder 4-Methyl-6-difluormethoxy-pyrimidin-2-yl oder 4-Methoxy-6-chlormethyl-pyrimidin-2-yl oder 4,6-Dimethoxy-s-triazin-2-yl, oder 4,6-Dimethoxy-pyrimidin-2-yl oder 4,6-Dimethylpyrimidin-2-yl oder 4-Methoxy-6-äthoxy-triazin-2-yl oder 4-Dimethylamino-6-methoxy-s-triazin-2-yl oder 4-Cyclopropyl-6-methoxy-s-triazin-2-yl oder 4-Methoxy-6-methoxymethylpyrimidin-2-yl oder 4-Methoxy-6-chlorpyrimidin-2-yl oder 1,5-Dimethyl-1,2,4-triazol-3-yl oder ein 5-Methoxy-1-methyl-1,2,4-triazol-3-yl Radikal ist.

6. Verfahren gemäss Anspruch 1, dadurch gekennzeichnet, dass man die Umsetzung in Acetonitril, Tetrahydrofuran, Dioxan, 1,2-Dimethoxyäthan, Dimethylformamid, N,N-Dimethyl-acetamid oder N-Methyl-pyrrolidon als Lösungsmittel durchführt.

7. Verfahren gemäss Anspruch 6, dadurch gekennzeichnet, dass man die Umsetzung in Acetonitril durchführt.

8. Verfahren gemäss Anspruch 1, dadurch gekennzeichnet, dass man die Gesamtmenge des Phenylcarbamat durch Säurezugabe aus dem Phenylcarbamatsalz zuerst gänzlich freisetzt und das Amin anschliessend zugibt.

9. Verfahren gemäss Anspruch 1, dadurch gekennzeichnet, dass man das Phenylcarbamat aus dem Phenylcarbamatsalz durch gleichzeitige Zugabe der hierzu nötigen Säure mit dem Amin freisetzt.

10. Verfahren gemäss Anspruch 9, dadurch gekennzeichnet, dass man das Amin als Salz einsetzt.

11. Verfahren gemäss Anspruch 1, dadurch gekennzeichnet, dass man zur Freisetzung des Sulfonyl-phenyl-carbamats oder zur Salzbildung mit den Aminen Methansulfonsäure oder Chlorwasserstoff verwendet.

12. Verfahren gemäss Anspruch 11, dadurch gekennzeichnet, dass man als Säure Methansulfonsäure verwendet.

13. Verfahren gemäss Anspruch 11, dadurch gekennzeichnet, dass man als Säure Chlorwasserstoff in Acetonitril gelöst verwendet.

14. Verfahren gemäss Anspruch 1, dadurch gekennzeichnet, dass man als Base Natrium- oder Kaliumhydroxyd, Natrium- oder Kaliumalkoholat oder tertiäre Amine verwendet.

15. Verfahren gemäss Anspruch 14, dadurch gekennzeichnet, dass man als Base Kaliumhydroxyd verwendet.

16. Verfahren gemäss Anspruch 14, dadurch gekennzeichnet, dass man als Base 1,8-Diazabicyclo(5,4,0)undec-7-en oder 1,5-Diazobicyclo-(4,3,0)non-5-en verwendet.

17. Verfahren gemäss Anspruch 16, dadurch gekennzeichnet, dass man das zur Salzbildung mit dem Sulfonamid eingesetzte tert. Amin unter Ausschluss von Wasser in ein vorgelegtes, aus dem Sulfonamid, aus Diphenylcarbonat und aus dem aprotischen Lösungsmittel bestehendes Gemisch tropft und das Phenylcarbamat-Salz bildet.

18. Verfahren gemäss Anspruch 16, dadurch gekennzeichnet, dass man die Basen nach der Umsetzung wiedergewinnt und rezykliert.

19. Verfahren gemäss Anspruch 8, dadurch gekennzeichnet, dass man die Umsetzung in einem einzigen Reaktionsgefäss durchführt.

## Claims

1. A process for producing sulfonylureas of the general formula

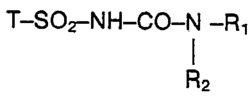 (I)

wherein

$R_1$ is hydrogen or $C_1$–$C_4$ alkyl,

$R_2$ is 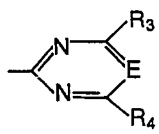 or 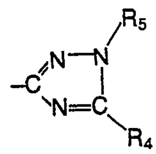 ,

E is =N– or =CH–,

$R_3$ is $C_1$–$C_4$-alkyl, $C_1$–$C_4$-alkoxy or halogen,

$R_4$ is $C_1$–$C_4$-alkyl, $C_3$–$C_6$-cycloalkyl, $C_1$–$C_4$-alkoxy, halogen, $C_1$–$C_4$-alkoxy-$C_1$–$C_4$-alkyl, halo-$C_1$–$C_4$-alkyl or halo-$C_1$–$C_4$-alkoxy.

$R_5$ is hydrogen or $C_1$–$C_4$-alkyl,

T is a substituted phenyl group

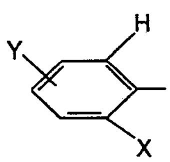 or 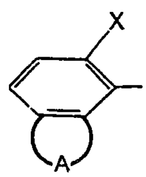 ,

Y is hydrogen or halogen,

X is hydrogen, halogen, nitro, $C_1$–$C_4$-alkyl, halo-$C_1$–$C_4$-alkyl, $C_2$–$C_4$-alkenyl, halo-$C_2$–$C_4$-alkenyl, $C_2$–$C_4$-alkynyl, $C_1$–$C_4$-alkoxy, halo-$C_1$–$C_4$-alkoxy, $C_1$–$C_4$-alkoxy-$C_1$–$C_4$-alkoxy, $C_1$–$C_4$-alkylthio, $C_1$–$C_4$-alkylsulfinyl, $C_1$–$C_4$-alkylsulfonyl, halo-$C_1$–$C_4$-alkylthio, $C_1$–$C_4$-alkylsulfonyloxy, phenyl, unsubstituted phenylsulfonyloxy or phenylsulfonyloxy mono- or optionally polysubstituted by $C_1$–$C_4$-alkyl, or is $C_1$–$C_4$-dialkylsulfamoyl, and

A is an unsubstituted or substituted bridge member which has 3 or 4 atoms and which contains 1 or 2 hetero atoms and forms, together with the carbon atoms to which it is bound, a nonaromatic 5- or 6-membered heterocycle, two oxygen atoms being separated by at least one carbon atom, and oxygen and sulfur atoms being linked together only when the sulfur is present as the –SO- or $-SO_2$-group,

by reacting a sulfonamide of the formula II

$$T-SO_2-NH_2 \quad (II),$$

in the presence of a base, with diphenyl carbonate to form a salt of a phenyl carbamate of the formula III

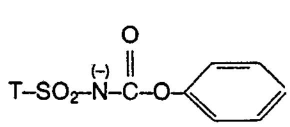 Me$^+$ (III)

wherein Me$^+$ is a cation of sodium or of potassium or of a tertiary amine, converting this salt into the free phenyl carbamate of the formula IIIa

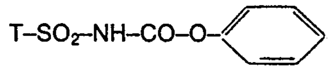 (IIIa) ,

and reacting this further with an amine of the formula IV

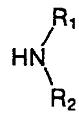 (IV),

wherein the reaction of the sulfonamide of the formula II with the diphenyl carbonate is performed, with the exclusion of water, in an aprotic solvent at 0–30 °C and, without isolation of an intermediate, there is subsequently liberated from the resulting salt of a phenyl carbamate of the formula III, by the addition of an anhydrous acid, the phenyl carbamate of the formula IIIa, and this is converted, by reaction with an amine of the formula IV at 20–150 °C, into a sulfonylurea of the formula I.

2. A process according to claim 1 for producing sulfonylureas of the general formula I

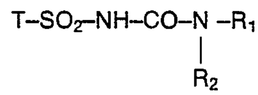 (I)

wherein

$R_1$ and $R_2$ have the meanings defined in claim 1,

T is a substituted phenyl group.

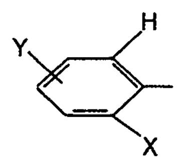 or 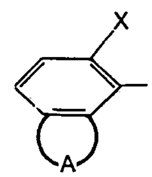 ,

and

X is hydrogen, halogen, $C_1$–$C_4$-alkyl, halo-$C_1$–$C_4$-alkyl, $C_1$–$C_4$-alkenyl, halo-$C_2$–$C_4$-alkenyl, $C_2$–$C_4$-alkynyl, $C_1$–$C_4$-alkoxy, halo-$C_1$–$C_4$-alkoxy, $C_1$–$C_4$-alkylthio, $C_1$–$C_4$-alkylsulfinyl, $C_1$–$C_4$-alkylsulfonyl, halo-$C_1$–$C_4$-alkylthio or $C_1$–$C_4$-dialkylsulfamoyl, and

A has the meaning defined in claim 1.

3. A process according to claim 1, wherein there are used the following sulfonamides of the formula II:

2-fluorobenzenesulfonamide,
2-chlorobenzenesulfonamide,
2-nitrobenzenesulfonamide,
2-methylbenzenesulfonamide,
2-trifluoromethylbenzenesulfonamide,
2-methoxybenzenesulfonamide,
2-difluoromethoxybenzenesulfonamide,
2-(2′-chloroethoxy)-benzenesulfonamide,
2-(2′-methoxyethoxy)-benzenesulfonamide,
2-n-propylthiobenzenesulfonamide,
2-methylsulfonylbenzenesulfonamide,
2-n-propylsulfonylbenzenesulfonamide,

2-methylsulfonyloxybenzylsulfonamide, 2-n-propylsulfonyloxybenzenesulfonamide, 2-dimethylsulfamoylbenzenesulfonamide, and 2,3-dihydrobenzofurano-7-ylsulfonamide.

4. A process accroding to claim 1, wherein there are preferably used sulfonamides of the formula IIa

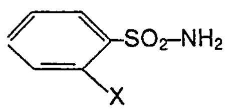

IIa

in which X has the meaning defined above.

5. A process according to claim 1, wherein there are used amines of the formula IV in which $R_1$ is hydrogen, and $R_2$ is: a 4-methyl-6-methoxy-s-triazin-2-yl, 4-methyl-6-methoxypyrimidin-2-yl, 4-methyl-6-difluoromethoxy-pyrimidin-2-yl, 4-methoxy-6-chloromethylpyrimidin-2-yl, 4,6-dimethoxy-s-triazin-2-yl, 4,6-dimethoxypyrimidin-2-yl, 4,6-dimethylpyrimidin-2-yl, 4-methoxy-6-ethoxy-triazin-2-yl, 4-dimethylamino-6-methoxy-s-triazin-2-yl, 4-cyclopropyl-6-methoxy-s-triazin-2-yl, 4-methoxy-6-methoxymethylpyrimidin-2-yl, 4-methoxy-6-chloropyrimidin-2-yl, 1,5-dimethyl-1,2,4-triazol-3-yl or 5-methoxy-1-methyl-1,2,4-triazol-3-yl radical.

6. A process according to claim 1, wherein the solvent in which the reaction is performed is acetonitrile, tetrahydrofuran, dioxane, 1,2-dimethoxyethane, dimethylformamide, N,N-dimethylacetamide or N-methylpyrrolidone.

7. A process according to claim 6, wherein the reaction is performed in acetonitrile.

8. A process according to claim 1, wherein the total amount of phenyl carbamate is firstly completely liberated from the phenyl carbamate salt by the addition of an acid, and the amine is subsequently added.

9. A process according to claim 1, wherein the phenyl carbamate is liberated from the phenyl carbamate salt by the simultaneous addition of the acid necessary for the purpose and the amine.

10. A process according to claim 9, wherein the amine is used in the form of a salt.

11. A process according to claim 1, wherein methansulfonic acid or hydrogen chloride is used for liberating the sulfonyl-phenyl carbamate or for salt formation with the amines.

12. A process according to claim 11, wherein the acid used is methanesulfonic acid.

13. A process according to claim 11, wherein the acid used is hydrogen chloride dissolved in acetonitrile.

14. A process according to claim 1, wherein the base used is sodium or potassium hydroxide, sodium or potassium alcoholate or tertiary amines.

15. A process according to claim 14, wherein the base used is potassium hydroxide.

16. A process according to claim 14, wherein the base used is 1,8-diazabicyclo(5,4,0)undec-7-ene or 1,5-diazobicyclo(4,3,0)non-5-ene.

17. A process according to claim 16, wherein the tertiary amine used for salt formation with the sulfonamide is added dropwise, with the exclusion of water, to a prepared mixture consisting of the sulfonamide, the diphenyl carbonate and the aprotic solvent to form the phenyl carbamate salt.

18. A process according to claim 16, wherein the bases, after the reaction, are recovered and recycled.

19. A process according to claim 8, wherein the reaction is performed in a single reaction vessel.

**Revendications**

1. Procédé de préparation de sulfonylurées de formule générale I

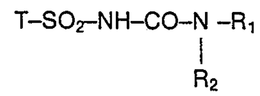

(I)

dans laquelle

$R_1$ représente H, un groupe alkyle en $C_1$–$C_4$,

$R_2$ représente

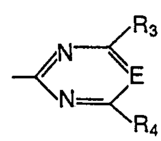

ou

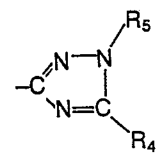

E représente =N– ou =CH–,

$R_3$ représente un groupe alkyle en $C_1$–$C_4$, alcoxy en $C_1$–$C_4$ ou un halogène,

$R_4$ représente un groupe alkyle en $C_1$–$C_4$, cycloalkyle en $C_3$–$C_6$, alcoxy en $C_1$–$C_4$, un halogène, un groupe (alcoxy en $C_1$–$C_4$)-alkyle en $C_1$–$C_4$, halogénoalkyle en $C_1$–$C_4$ ou halogénoalcoxy en $C_1$–$C_4$,

$R_5$ représente H ou un groupe alkyle en $C_1$–$C_4$,

T représente un groupe phényle substitué

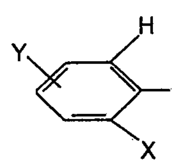

ou

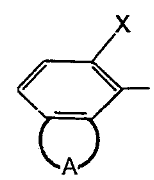

Y représente H ou un halogène,

X représente H, un halogène, un groupe nitro, alkyle en $C_1$–$C_4$, halogénoalkyle en $C_1$–$C_4$, alcényle en $C_2$–$C_4$, halogénoalcényle en $C_2$–$C_4$, alcynyle en $C_2$–$C_4$, alcoxy en $C_1$–$C_4$, halogénoalcoxy en $C_1$–$C_4$, (alcoxy en $C_1$–$C_4$)-alcoxy en $C_1$–$C_4$, alkylthio en $C_1$–$C_4$, alkylsulfinyle en $C_1$–$C_4$, alkylsulfonyle en $C_1$–$C_4$, halogénoalkylthio en $C_1$–$C_4$, alkylsulfonyloxy en $C_1$–$C_4$, phényle, phénylsulfonyloxy non substitué ou phénylsulfonyloxy mono- ou le cas échéant polysubstitué par des groupes alkyle en $C_1$–$C_4$ ou di-(alkyle en $C_1$–$C_4$)-sulfamoyle et

A représente un pont à 3 ou 4 atomes, éventuellement substitué, contenant 1 ou 2 hétéro-atomes et formant avec les atomes de carbone auquel il est relié un hétérocycle non aromatique à 5 ou 6 chaînons dans lequel deux atomes d'oxygène sont séparés par au moins un atome de carbone, et les atomes d'oxygène et les atomes de soufre ne peuvent être reliés entre eux que lorsque le soufre est à l'état de groupe –SO– ou $-SO_2-$, par réaction d'un sulfonamide de formule II,

$T{-}SO_2{-}NH_2$ II

en présence d'une base, avec le carbonate de diphényle, la réaction donnant un sel d'un carbamate de phényle de formule III

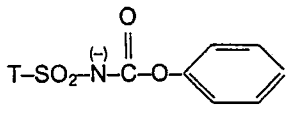 $Me^+$ (III),

dans laquelle $Me^+$ représente un cation sodium ou potassium ou un cation d'amine tertiaire, qu'on convertit en le carbamate de phényle libre de formule IIIa

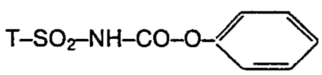 (IIIa)

qu'on fait ensuite réagir avec une amine de formule IV,

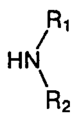 (IV),

ce procédé se caractérisant en ce que l'on fait réagir le sulfonamide de formule II avec le carbonate de diphényle à l'abri de l'humidité dans un solvant apronique à des températures de 0 à 30 °C et, sans isoler le produit intermédiaire, on libère ensuite, à partir du sel de carbamate de phényle de formule III ainsi formé, par addition d'un acide anhydre, le carbamate de phényle de formule IIIa qu'on convertit par réaction avec une amine de formule IV à des températures de 20 à 150 °C en une sulfonylurée de formule I.

2. Procédé selon la revendication 1 pour la préparation de sulfonylurées de formule générale I

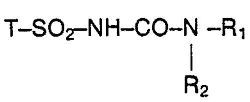 (I)

dans laquelle $R_1$ et $R_2$ ont les significations indiquées dans la revendication 1,

T représente un groupe phényle substitué

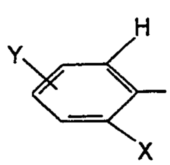 ou 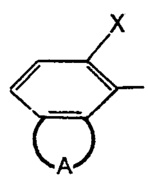

et

X représente H, un halogène, un groupe alkyle en $C_1$–$C_4$, halogénoalkyle en $C_1$–$C_4$, alcényle en $C_1$–$C_4$, halogénoalcényle en $C_2$–$C_4$, alcynyle en $C_2$–$C_4$, alcoxy en $C_1$–$C_4$, halogénoalcoxy en $C_1$–$C_4$, alkylthio en $C_1$–$C_4$, alkylsulfynyle en $C_1$–$C_4$, alkylsulfonyle en $C_1$–$C_4$, halogénoalkylthio en $C_1$–$C_4$ ou di-(alkyle en $C_1$–$C_4$)-sulfamoyle, et

A a les significations indiquées dans la revendication 1.

3. Procédé selon la revendication 1, caractérisé en ce que l'on met en œuvre les sulfonamides suivants, répondant à la formule II:
2-fluoro-benzènesulfonamide,
2-chloro-benzènesulfonamide,
2-nitro-benzènesulfonamide,
2-méthyl-benzènesulfonamide,
2-trifluorométhyl-benzènesulfonamide,
2-méthoxy-benzènesulfonamide,
2-difluorométhoxy-benzènesulfonamide,
2-(2'-chloréthoxy)-benzènesulfonamide,
2-(2'-méthoxy-éthoxy)-benzènesulfonamide,
2-n-propylthio-benzènesulfonamide,
2-méthylsulfonyl-benzènesulfonamide,
2-n-propylsulfonyl-benzènesulfonamide,
2-méthylsulfonyloxy-benzylsulfonamide,
2-n-propylsulfonyloxy-benzènesulfonamide,
2-diméthylsulfamoyl-benzènesulfonamide,
2,3-dihydro-benzofuranne-7-yl-sulfonamide.

4. Procédé selon la revendication 1, caractérisé en ce que l'on met en œuvre de préférence les sulfonamides de formule IIa

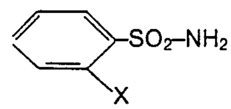 IIa

dans laquelle X a les significations indiquées ci-dessus.

5. Procédé selon la revendication 1, caractérisé en ce que l'on utilise des amines de formule IV dans laquelle $R_1$ représente l'hydrogène et $R_2$ un groupe 4-méthyl-6-méthoxy-s-triazine-2-yle ou le 4-méthyl-6-méthoxy-pyrimidine-2-yle ou 4-méthyl-6-difluorométhoxy-pyrimidine-2-yle ou 4-méthoxy-6-chlorométhyl-pyrimidine-2-yle ou 4,6-diméthoxy-s-triazine-2-yle ou 4,6-diméthoxy-pyrimidine-2-yle ou 4,6-diméthylpyrimidine-2-yle ou 4-méthoxy-6-éthoxy-triazine-2-yle ou 4-diméthylamino-6-méthoxy-s-triazine-2-yle ou 4-cyclopropyl-6-méthoxy-s-triazine-2-yle ou 4-méthoxy-6-méthoxy-méthyl-pyrimidine-2-yle ou 4-méthoxy-6-chloropyrimidine-2-yle ou 1,5-diméthyl-1,2,4-triazole-3-yle ou 5-méthoxy-1-méthyl-1,2,4-triazole-3-yle.

6. Procédé selon la revendication 1, caractérisé en ce que l'on effectue la réaction dans l'acétonitrile, le tétrahydrofuranne, le dioxanne, le 1,2-diméthoxyéthane, le diméthylformamide, le N,N-diméthylacétamide ou la N-méthylpyrrolidone servant de solvant.

7. Procédé selon la revendication 6, caractérisé en ce que l'on effectue la réaction dans l'acétonitrile.

8. Procédé selon la revendication 1, caractérisé en ce que l'on libère d'abord en totalité tout la carbamate de phényle à partir du sel de carbamate de phényle par addition d'un acide et on ajoute ensuite l'amine.

9. Procédé selon la revendication 1, caractérisé en ce que l'on libère le carbamate de phényle à partir du sel de carbamate de phényle par addition simultanée de l'acide nécessaire à cet effet et de l'amine.

10. Procédé selon la revendication 9, caractérisé en ce que l'on met en œuvre l'amine à l'état de sel.

11. Procédé selon la revendication 1, caractérisé en ce que, pour la libération du sulfonylcarbamate de phényle ou pour la formation de sels avec les amines, on utilise l'acide méthane-sulfonique ou l'acide chlorhydrique.

12. Procédé selon la revendication 11, caractérisé en ce que l'acide utilisé est l'acide méthane-sulfonique.

13. Procédé selon la revendication 11, caractérisé en ce que l'acide utilisé est l'acide chlorhydrique en solution dans l'acétonitrile.

14. Procédé selon la revendication 1, caractérisé en ce que la base utilisée est l'hydroxyde de sodium ou de potassium, un alcoolate de sodium ou de potassium ou une amine tertiaire.

15. Procédé selon la revendication 14, caractérisé en ce que la base utilisée est l'hydroxyde de potassium.

16. Procédé selon la revendication 14, caractérisé en ce que la base utilisée est le 1,8-diazabicyclo(5,4,0)undéca-7-ène ou le 1,5-diazabicyclo-(4,3,0)nona-5-ène.

17. Procédé selon la revendication 16, caractérisé en ce que l'amine tertiaire utilisée pour la formation d'un sel avec le sulfonamide est introduite goutte à goutte à l'abri de l'humidité dans un mélange consistant en le sulfonamide, le carbonate de diphényle et le solvant apronique, avec formation du sel de carbamate de phényle.

18. Procédé selon la revendication 16, caractérisé en ce que, après la réaction, les bases sont récupérées et recyclées.

19. Procédé selon la revendication 8, caractérisé en ce que l'on effectue la réaction dans un seul récipient de réaction.